# EUROPEAN PATENT APPLICATION

(11) **EP 4 309 687 A1**
(43) Date of publication of application: **24.01.2024**
(21) Application number: 22784093.1
(22) Date of filing: 07.04.2022
(51) Int. Cl.: A61L 31/02, A61L 31/08, A61L 31/12, C23C 14/00, C23C 28/00, C25D 3/04, C25D 3/50, C25D 3/54, C25D 3/56, C25D 9/04

(54) **MEDICAL DEVICE**

(30) Priority: 08.04.2021 CN 202110376269
(71) Applicant: Shanghai MicroPort Medical (Group) Co., Ltd., Shanghai 201203 (CN)
(72) Inventor: WANG, Jiaojiao, Shanghai 201203 (CN); SUN, Can, Shanghai 201203 (CN); ZHOU, Qi, Shanghai 201203 (CN); YAO, Yao, Shanghai 201203 (CN); LI, Junfei, Shanghai 201203 (CN)
(74) Representative: Marks & Clerk LLP
(86) International application number: PCT/CN2022/085545
(87) International publication number: WO 2022/214027

(57) **Abstract**

The present disclosure relates to a medical device. The medical device comprises a matrix and a first coating coated on the matrix. The matrix contains a metal element X having a content of ≥5wt%; the first coating is at least one of an elemental metal layer of the metal element X, an alloy layer of the metal element X, and a metal-ceramic layer of the metal element X; and the first coating does not contain nickel and cobalt. The medical device has good use safety, reliability, and biocompatibility, so that a product can meet a use requirement of large plastic deformation, and has good reliability during use. The medical device can particularly meet requirements of product with large plastic deformation during use, such as medical implant devices.

## Description

### TECHNICAL FIELD

The present disclosure relates to the field of medical technologies, and particularly to a medical device.

### BACKGROUND

Biomedical metal materials have been widely used in clinical medicine for their good mechanical properties, ease of processing and reliability, and their importance is equal to that of biomedical polymer materials, occupying a very important proportion in the whole biomedical material applications. The development of medical metal materials focuses on continuously improving use safety, reliability and biocompatibility.

After many years of clinical application, there are still many problems on medical metal materials, the main problem is still biocompatibility. Biocompatibility means does not cause adverse reactions to organism's tissues, blood, etc. Since metal materials contain many alloying elements, after implantation into the human body, metal ions may dissolve due to corrosion, wear and tear, and the like, which in turn triggers some biological reactions in cells and tissue fluids, such as tissue reactions, blood reactions and systemic reactions, manifested as edema, thromboembolism, infections, tumors, and other phenomena. As is well-known, in addition to being highly toxic and allergic to the human body, a nickel ion may induce mutations in organisms and even cancer. Recent studies have found that the release of a cobalt ion can also cause necrosis of cells and tissues and allergic skin reactions. The most widely used medical metal load-bearing implant materials in clinical applications in the current market, such as stainless steel, cobalt alloys, and nickel-titanium shape memory alloys, all contain nickel alloy elements; at the same time, the cardiovascular stent materials that dominate the market, in addition to containing nickel alloy elements, cobalt ions released from the substrate also exacerbate the problem of their biosafety.

In summary, when developing new medical metal materials, strict control of the metal element X thereof is needed, preferably with less or no use of alloying elements that are toxic and allergic to the human body. Technical methods generally employed to improve biosafety include alloying, improving corrosion resistance, improving the degree of finish, and surface coating. The fundamental solution to the problem is alloying and surface coating method. Because the alloying method needs to be considered from composition design, and through multiple processes including alloy melting, heat treatment and post-processing, the cycle time is long, the investment is large, and the risk is high. Surface coating technology for implant materials is an effective method that can address the dissolution of nickel and cobalt ions.

### SUMMARY

Accordingly, it is necessary to provide a medical device with good safety, reliability and biocompatibility.

A medical device includes a substrate and a first coating wrapping the substrate, the substrate contains a metal element X with a content of ≥5wt%, the first coating includes at least one of an elemental metal layer of the metal element X, an alloy layer of the metal element X, and a metal-ceramic layer of the metal element X, and the first coating does not contain nickel and cobalt.

In some of embodiments, the first coating is a stack layer formed by the elemental metal layer and at least one of the alloy layer and the metal-ceramic layer;

in the first coating, the elemental metal layer is an inner layer close to the substrate, at least one of the alloy layer and the metal-ceramic layer is an outer layer away from the substrate.

In some of embodiments, the elemental metal layer is a passivated metal coating when the first coating is the elemental metal layer of the metal element X; or
the elemental metal layer is located between the substrate and the metal-ceramic layer when the first coating is the stack layer formed by the elemental metal layer and the metal-ceramic layer; or
the elemental metal layer is located between the substrate and the alloy layer when the first coating is the stack layer formed by the elemental metal layer and the alloy layer.

In some of embodiments, a difference of self-corrosion potential between the first coating and the substrate is within 100 mV.

In some of embodiments, a material of the metal-ceramic layer is at least one of oxide, carbide, nitride, silicide, and boride of the metal element X.

In some of embodiments, the metal element X is Cr, W, Mo or any one of noble metal element.

In some of embodiments, the content of the metal element X in the substrate ranges from 7 wt% to 60 wt%.

In some of embodiments, the substrate is a substrate capable of plastic deformation.

In some of embodiments, a material of the substrate is selected from the group consisting of stainless steel, cobalt-based alloy, titanium alloy, noble metal alloy, and nickel-titanium shape memory alloy.

In some of embodiments, the metal element X is one of Cr, W, Mo or Pt.

In some of embodiments, the first coating contains the metal-ceramic layer, the medical device further includes a second coating provided on the metal-ceramic layer;
wherein the metal-ceramic layer is a carbide of the metal element X, and the second coating is a carbon layer; or
the metal-ceramic layer is a silicide of the metal element X, and the second coating is a silicon layer.

In some of embodiments, a difference of self-corrosion potential between any two adjacent layers of layer structures with different materials of the medical device is within 100 mV.

In some of embodiments, thicknesses of the elemental metal layer, the alloy layer, and the metal-ceramic layer range from 10 nm to 500 nm.

In some of embodiments, the metal element X is not element titanium.

In some of embodiments, the medical device further includes a medicine active layer located on a surface of the first coating.

In some of embodiments, the first coating is formed with a medicine-carrying recess, and the medicine active layer is located within the medicine-carrying recess.

According to the aforementioned medical device in the present disclosure, the first coating with a specific material is formed on the substrate, the first coating contains the metal element X with the content of ≥5 wt% in the substrate, and the metal element X is an element except nickel and cobalt. As such, the first coating with the same metal element X as the substrate is used on the substrate, on the one hand, the problem of cracking due to the large difference in properties such as ductility between the coating and the substrate can be avoided, and the first coating and the substrate can have excellent bonding force and ductility, such that the product can meet the use requirement of large plastic deformation, and has good reliability during use, particularly can meet the requirement of the products with large plastic deformation during use, such as medical implant devices. On the other hand, the first coating wrapping the substrate does not contain nickel and cobalt, which can achieve the effect of isolating or significantly reducing the dissolution of sensitized and carcinogenic alloy elements such as nickel and cobalt contained in the substrate, and has better use safety and biocompatibility.

### BRIEF DESCRIPTION OF THE DRAWINGS

FIG. 1 is a schematic view of a vascular stent according to an embodiment;
FIG. 2 is a scanning electron microscope image of a stent product made in comparative example 2 after expansion;
FIG. 3 is the scanning electron microscope image of a stent product made in comparative example 4 after expansion;
FIG. 4 is the scanning electron microscope image of a stent product made in comparative example 5 after expansion;
FIG. 5 is the scanning electron microscope image of a stent product made in comparative example 6;
FIG. 6 is the scanning electron microscope image of a stent product made in comparative example 7 expanded to nominal size;
FIG. 7 is the scanning electron microscope image of a stent product made in comparative example 8 expanded to extreme post-expansion size;
FIG. 8 is the scanning electron microscope image of a stent product made in comparative example 9 after simulated traversal;
FIG. 9 is the scanning electron microscope image of a stent product made in example 4 expanded to nominal size;
FIG. 10 is the scanning electron microscope image of a stent product made in example 8 expanded to extreme post-expansion size.

### DETAILED DESCRIPTION

In order to facilitate the understanding of the present disclosure, the present disclosure will be described more fully hereinafter with reference to the related accompanying drawings. Preferable embodiments are presented in the accompanying drawings. However, the present disclosure may be embodied in many different forms and is not limited to the embodiments described herein. Rather, these embodiments are provided so that the understanding of the content of the present disclosure will be more thorough.

It should be understood that when an element is defined as "located on" another element, it is either directly on an element or there may be an intermediate element between them.

All technical and scientific terms used herein have the same meaning as commonly understood by one of ordinary skill in the art to which the present disclosure applies, unless otherwise defined. The terms used in the specification of the present disclosure herein are for the purpose of describing specific embodiments only and are not intended to limit the present disclosure. The term "and/or" used herein includes any and all combinations of one or more of the associated listed items.

In the description of the present disclosure, it should be understood that the terms "first" and "second" are used for descriptive purposes only, and cannot be understood to indicate or imply relative importance or implicitly indicate the number of technical features indicated. Therefore, the defined "first" and "second" features may explicitly or implicitly include one or more of the features. In the description of the present disclosure, "plurality" means at least two, such as two or more than two, unless otherwise specifically defined.

An embodiment of the present disclosure provides a medical device including a substrate and a first coating wrapping the substrate.

The substrate contains a metal element X with a content of ≥5 wt%. The first coating is at least one of an elemental metal layer of the metal element X, an alloy layer of the metal element X, and a metal-ceramic layer of the metal element X. And the first coating does not contain nickel and cobalt. In other words, the metal element X is not nickel and cobalt, and the alloy layer does not contain nickel and cobalt either.

In the medical device according to an embodiment of the present disclosure, the first coating with a specific material is formed on the substrate, the first coating contains the metal element X with the content of ≥5 wt% in the substrate, and the metal element X is an element except nickel and cobalt. As such, the first coating with the same metal element X as the substrate is used on the substrate, on the one hand, the problem of cracking due to the large difference in properties such as ductility between the coating and the substrate can be avoided, and the first coating and the substrate can have excellent bonding force and ductility, such that the product can meet the use requirement of large plastic deformation, and has good reliability during use, particularly can meet the requirement of products with large plastic deformation during use, such as medical implant devices. On the other hand, the first coating wrapping the substrate does not contain nickel and cobalt, which can achieve the effect of isolating or significantly reducing the dissolution of sensitized and carcinogenic alloy elements such as nickel and cobalt contained in the substrate, and has better use safety and biocompatibility.

Meanwhile, the first coating also improves the corrosion resistance property of the substrate material, which in turn can prolong the service life of the medical device to a certain extent, thus bringing great convenience to clinical applications.

In some of embodiments, the medical device is a medical implant device or a medical interventional device. It should be understood that the medical device according to an embodiment of the present disclosure is particularly suitable for, but not only suitable for, the medical implant devices with large plastic deformation during use, and is also suitable for products such as medical interventional devices. In other words, the substrate may be, but is not limited to, a substrate capable of plastic deformation.

It is worth noting that the medical implant device includes but not limited to vascular stents, coronary stents, heart valve stents, cardiac pacemakers, venous filters, and metal bone nails, metal bone plates, and artificial joints. The medical interventional device includes but not limited to intravascular catheters, guidewires and sheaths, and embolization devices.

Taking a cardiovascular stent in medical implantable device as an example, in general, the cardiovascular stent has at least 30% of plastic deformation during implantation and expansion. During this process, the coating on the cardiovascular stent with poor bonding force and ductility is susceptible to large-scale cracking or peeling and falling off from the substrate. If the coating falls off, it may lead to serious consequences such as blood clots and death. In order to make the coating not only have large plastic deformation along with the substrate after compressed implantation of the cardiovascular stent, but also can prevent or significantly reduce the dissolution of sensitized and carcinogenic alloy elements such as nickel and cobalt, it is required to have good ductility and bonding force between the coating and the substrate. It has been verified by experiments that the coating and the substrate of the medical device according to an embodiment of the present disclosure meet the requirement of good bonding force therebetween, and thereby achieving the object.

In the medical device according to an embodiment of the present disclosure, the dissolution of sensitized and carcinogenic alloy elements, such as nickel and cobalt, is effectively reduced by surface coating method, which provides a cost-effective and efficient method to improve the biosafety of medical devices, particularly for medical implant devices.

As described above, the first coating is at least one of the elemental metal layer of the metal element X, the alloy layer of the metal element X, and the metal-ceramic layer of the metal element X. Therefore, a metallic bond, an ionic bond, a covalent bond or a molecular bond may be formed between the first coating and the substrate, such that the first coating and the substrate have good bonding force therebetween. For example, when the elemental metal layer wraps the substrate, and a metallic bond may be formed between the elemental metal layer and the substrate.

In some of embodiments, the metal element X may be chromium (Cr), or tungsten (W), or molybdenum (Mo), or any one of noble metal element, where the noble metal element includes gold (Au), silver (Ag), mercury (Hg), platinum (Pt), palladium (Pd), iridium (Ir), rhodium (Rh), ruthenium (Ru), and osmium (Os).

Further, the metal element X is not element titanium.

It can be understood that on the basis that the metal element X is not nickel and cobalt and the content thereof in the substrate is ≥5 wt%, theoretically, the higher the content of the metal element X in the substrate, the better the bonding force of the coating to the substrate. Optionally, in some of embodiments, the content of the metal element X in the substrate is ≥7 wt%. Optionally, in some of embodiments, the content of the metal element X in the substrate is ≥9 wt%.

Further considering the selection of material for the substrate of the medical device, further, in some of embodiments, the content of the metal element X in the substrate ranges from 7 wt% to 60 wt%; optionally, ranges from 9 wt% to 60 wt%; preferably, ranges from 9 wt% to 40 wt%; more preferably, ranges from 9 wt% to 35 wt%.

It can be understood that in some of embodiments, the metal element X may also be an alloy composed of two or more metals.

In some of embodiments, the first coating is the elemental metal layer of the metal element X. As such, the elemental metal layer may be passivated to improve the compactness of a passivation film on surface of a pure metal functional coating, which in turn further improves the surface corrosion resistance of the elemental metal layer.

It can be understood that the elemental metal layers can be passivated under natural conditions or passivated by a nitric acid solution. If a naturally passivated oxide layer of the elemental metal layer is relatively thin, the elemental metal layer may also be passivated by the nitric acid solution to further improve the corrosion resistance of the coating. For example, the elemental metal layer is passivated by immersing in a nitric acid solution with a concentration between 40 wt% and 60 wt%. Further, the passivation condition includes, for example, a passivation temperature between 20°C and 30°C, and a passivation time between 30 min and 60 min.

It can be understood that the above alloy layer may be a metal alloy layer formed by at least two metals including the metal element X. In some embodiments, the metal element of the alloy layer is selected from at least two of the group consisting Cr, W, Mo, and noble metal element.

In some of embodiments, the first coating is the alloy layer of the metal element X.

It can be understood that the metal-ceramic layer includes but not limited to metal oxides, metal carbides, metal nitrides, metal silicates and metal borides. In some of embodiments, the material of the above metal-ceramic layer is at least one of oxide, carbide, nitride, silicide, and boride of the above metal element X. Preferably, the material of the metal-ceramic layer described above is at least one of oxide, carbide, nitride, and silicide of the above metal element X.

In some of embodiments, the first coating is the metal-ceramic layer of the metal element X.

In some of embodiments, the first coating is a stack layer formed by the elemental metal layer and at least one of the alloy layer and the metal-ceramic layer (or called composite coating). In the first coating, the elemental metal layer is an inner layer close to the substrate, at least one of the alloy layer and the metal-ceramic layer is an outer layer away from the substrate. The bonding force between the first coating and the substrate can be improved due to better bonding force between the elemental metal layer and the substrate. Using the elemental metal layer with good ductility as the inner layer or a transition layer can significantly improve the ductility of the composite coating, and can be subjected to large plastic deformation along with the substrate without cracking failure during compression and expansion deformation of the medical device.

Further, the elemental metal layer is in direct contact with the substrate, such that the first coating and the substrate are bonded by the metallic bond, which has a better bonding force.

In some embodiments, the first coating is a stack layer formed by the elemental metal layer and the metal-ceramic layer, and the elemental metal layer is located between the substrate and the metal-ceramic layer.

Since the strength and the hardness of the metal-ceramic layer is high but the ductility thereof is poor, the metal-ceramic layer is located on the outer layer. On the basis of ensuring good ductility of the inner elemental metal layer, the first coating can exhibit high strength and hardness, which can meet requirements of orthopedic implant devices with high hardness and high wear resistance.

In some other embodiments, the first coating is a stack layer formed by the elemental metal layer and the alloy layer, and the elemental metal layer is located between the substrate and the alloy layer. As such, strong metallic bonds are formed between the elemental metal layer and the substrate, and between the elemental metal layer and the alloy layer, resulting in high bonding strength. Good plastic deformation capability of the functional coating can also be ensured while ensuring excellent bonding force. The metallic bond is not directional, so it will not be destroyed when the relative movement of atomic positions occurs by subjecting to external forces.

In some other embodiments, the first coating may be a stack layer sequentially formed by the elemental metal layer, the alloy layer, and the metal-ceramic layer. In the first coating, the elemental metal layer is the inner layer, the alloy layer is the middle layer, and the metal-ceramic layer is the outer layer.

It can be understood that in some other embodiments, the first coating may also be a stack layer formed by the alloy layer and the metal-ceramic layer, and the alloy layer is located between the substrate and the metal-ceramic layer.

In some of embodiments, the material of the substrate is selected from the group consisting of stainless steel, cobalt-based alloy, titanium alloy, noble metal alloy, and nickel-titanium shape memory alloy. It can be understood that the substrate may be nickel or cobalt containing material. Further, the noble metal alloy includes platinum-chromium alloy. The cobalt-based alloy includes cobalt-chromium alloy, such as L605, where the metal element X may be Cr or W; it also includes cobalt-chromium-molybdenum alloy, such as MP35N and ASTM F75, where the metal element X may be Cr or Mo.

In some of embodiments, when the material of the substrate is selected from the group consisting of stainless steel, cobalt-based alloy, and platinum-chromium alloy, the metal element X may be Cr. It can be understood that other suitable metal elements may also be selected as the metal element X, and metal element X may be specifically selected according to the material of the substrate.

In some of embodiments, the material of the substrate is L605 alloy, MP35N, 316L or PtCr alloy, the elemental composition of each substrate material employed in examples or comparative examples of the present disclosure is shown in Table 1 below.

**Table 1**

| Substrate material | Co | Cr | KV | Ni | Fe | Pt | Mo | Ti | Mn | Si | S | C | P |
|---|---|---|---|---|---|---|---|---|---|---|---|---|---|
| | Unit: wt% | | | | | | | | | | | | |
| L605 | Residual | 19-21 | 14-16 | 9-11 | <3.0 | / | / | / | 1.0-2.0 | <0.4 | <0.03 | 0.05-0.15 | <0.04 |
| MP35N | Residual | 19-21 | / | 33-37 | <1 | / | 9-10.5 | <1 | / | / | / | / | / |
| PtCr alloy | / | 17.5-18.5 | / | 8.5-9.5 | Residual | 32.5-33.5 | 2.43-2.83 | / | <0.05 | <0.1 | <0.0007 | 0.003-0.023 | <0.01 |
| 316L | / | 17-19 | / | 13-15 | Residual | / | 2.25-3.0 | / | <2.0 | <0.75 | <0.01 | <0.03 | <0.025 |
| Nickel-titanium | / | / | / | 54.5-57 | | / | / | 43-45.5 | / | / | / | / | / |
| Cobalt-chromium-molybdenum alloy ASTM F75 | Residual | 27-30 | <0.2 | <0.5 | <0.75 | / | 5-7 | <0.1 | <1 | <1 | <0.01 | <0.35 | <0.02 |

In some of embodiments, the material of the substrate is L605 alloy, the metal element X is Cr or W.

In some of embodiments, the material of the substrate is MP35N alloy or cobalt-chromium-molybdenum alloy ASTM F75, the metal element X is Cr or Mo.

In some of embodiments, the material of the substrate is 316L alloy, the metal element X is Cr.

In some of embodiments, the material of the substrate is PtCr alloy, the metal element X is Cr or Pt.

In other words, the material of the substrate is L605, MP35N, 316L, or PtCr alloy, the metal element X may be Cr.

In some of embodiments, thicknesses of the elemental metal layer, the alloy layer, and the metal-ceramic layer range from 10 nm to 500 nm; preferably, range from 10 nm to 300 nm; more preferably, range from 10 nm to 200 nm; more preferably, range from 10 nm to 100 nm; more preferably, range from 10 nm to 80 nm; more preferably, range from 30 nm to 80 nm.

By optimizing the selection of the thickness of each coating, it can be further ensured that the first coating is completely wrapped on all surfaces of the substrate without defects such as penetrating holes, and the release of nickel and cobalt ions from the substrate can be effectively avoided.

In the first coating, the thickness of the coating in direct contact with the substrate as the inner layer may range from 10 nm to 80 nm; preferably, range from 10 nm to 60 nm; particular preferably, range from 10 nm to 50 nm.

In some of embodiments, the total thickness of the first coating ranges from 10 nm to 30 nm, which is the relatively optimal thinnest thickness that can completely wrap the substrate, and can wrap the substrate even after the substrate is largely plastically deformed, and can ensure a low degree of dissolution of harmful ions.

In some of embodiments, the total thickness of the first coating ranges from 30 nm to 100 nm, which enables the first coating to be deformed in good coordination with the substrate, to have good bonding force and ductility, and to wrap the substrate even after the substrate is largely plastically deformed. The degree of dissolution of harmful ions is lower in this embodiment comparing to the embodiment where the total thickness of the first coating ranges from 10 nm to 30 nm (other conditions remain unchanged).

In some of embodiments, the total thickness of the first coating ranges from 100 nm to 300 nm. The first coating in this embodiment can be well deformed in coordination with the substrate, and have optimal bonding force and ductility, such that the first coating can wrap the substrate even after the substrate is largely plastically deformed. Compared to other embodiments, the degree of dissolution of harmful ions is lowest in this embodiment.

In some of embodiments, the total thickness of the first coating ranges from 300 nm to 500 nm. In this embodiment, the coating of the first coating is relatively large, after plastic deformation of the substrate, the first coating is susceptible to crack, which increases the tendency for harmful ions to dissolve.

In some of embodiments, any coating in the first coating is a nanometer-coating. The nanometer-sized functional coating has an activating catalytic effect on the substrate material, which is beneficial to further enhance the bonding force between the interface of the substrate and the coating, thus effectively solving the problem of the medical device having a falling off or a large-area cracking of the surface coating when the medical device undergoes a large plastic deformation. In addition, compared with the medical implant device with a micron-level coating or a larger-sized coating, the nanometer-coating has high compactness, which can reduce or effectively avoid the dissolution or precipitation of sensitized and carcinogenic nickel and cobalt elements, resulting in better biocompatibility and safety as a medical implant device, and expanding the scope of people applicable to the device.

The inventors of the present disclosure have found that, good bonding force between two adjacent layers of the coating can be further ensured by controlling the difference of self-corrosion potential between any two adjacent layers of layer structures with different materials within a reasonable range. Compared with other protective coatings with high self-corrosion potential, the risk of galvanic effect between the substrate and the coating in possible coating failure modes, such as cracking or falling off, can be reduced to a great extent. The layer structure between any two adjacent layers with different materials of the medical device includes the layer structure between the substrate and the coating in direct contact therewith, the layer structure between any two adjacent layers with different materials within the first coating when the first coating is the stack layer, and the layer structure between the second coating and the first coating as mentioned later, and the like.

Preferably, the difference of self-corrosion potential between any two adjacent layers of layer structures with different materials of the medical device is within 100 mV; more preferably, the difference of self-corrosion potential between any two adjacent layers of layer structures with different materials of the medical device is within 80 mV; even more preferably, the difference of self-corrosion potential between any two adjacent layers of layer structures with different materials of the medical device is within 60 mV; and particular preferably, the difference of self-corrosion potential between any two adjacent layers of layer structures with different materials of the medical device is within 50 mV; most preferably, the difference of self-corrosion potential between any two adjacent layers of layer structures with different materials of the medical device is within 30 mV.

In some of embodiments, the first coating contains the metal-ceramic layer. Further, the first coating also includes the second coating provided on the metal-ceramic layer. Further, the metal-ceramic layer is the carbide of the metal element X or the silicide of the metal element X, and the second coating is the silicon layer. Or, further, the metal-ceramic layer is the carbide of the metal element X, and the second coating is the carbon layer.

As such, by forming the silicon layer or the carbon layer on the first coating as the second coating, on the one hand, the second coating has good bonding force with the metal-ceramic layer, and on the other hand, the second coating can be avoided to contain metal elements, and the dissolution of trace metal elements from the metal-ceramic layer can be avoided.

The thickness of the second coating ranges from 30 nm to 500 nm. Preferably, the thickness of the second coating ranges from 30 nm to 200 nm; preferably, the thickness of the second coating ranges from 50 nm to 150 nm; preferably, the thickness of the second coating ranges from 50 nm to 150 nm.

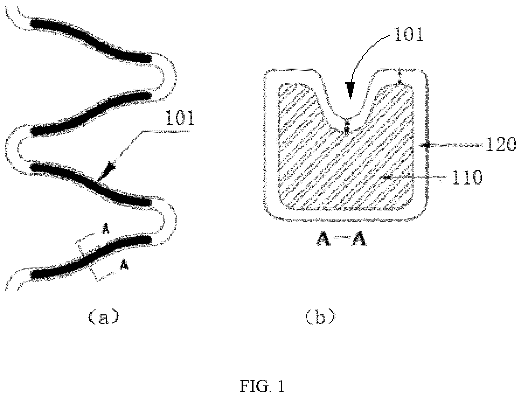

The surface of the medical device such as a vascular stent may be smooth or rough, may be provided with a groove or without a groove, may be provided with a hole or without a hole, or may include, but not limited to any other recesses or micro-pores that can carry medicine. In some of embodiments, the first coating is formed with a medicine-carrying recess. As the vascular stent shown in FIG. 1, the stent is consisted of one or more wavy rods. The wavy rod is consisted of a substrate 110 and a first coating 120, and the first coating 120 is provided with a recess 101. In FIG. 1, (a) shows a schematic view of the wavy rod, and (b) shows a schematic cross-sectional view of the wavy rod along the A-A line.

In some of embodiments, the medical device further includes the medicine active layer provided on the surface of the first coating. Further, the medicine active layer may be provided within the medicine-carrying recess.

Further, the medicine in the medicine active layer includes medicine for treating cardiovascular and cerebrovascular diseases, medicine for oncological diseases, medicine for treating intestinal diseases, medicine for treating urinary tract diseases, and the like. Further, wherein the medicine includes but not limited to, at least one of Paclitaxel, Docetaxel, Copper aspirinate, Tacrolimus, Hydroxy camptothecin, Vinblastine, Doxorubicin, Rapamycin and Rapamycin derivatives.

An embodiment of the present disclosure also provides a method for preparing the medical device, including the following steps:
Providing a substrate; and
forming a first coating wrapping the substrate.

It can be understood that when the first coating is at least two of the elemental metal layer, the alloy layer and the metal-ceramic layer, the first coating is formed stepwise.

As described above, in some of embodiments, the medical device further includes a second coating.

In some of embodiments, the method of forming the first coating and the second coating may be: physical or chemical surface modification methods such as physical vapor deposition, thermal spraying, pulsed laser thin film deposition, magnetron sputtering deposition, evaporation plating, ion plating, chemical plating, electrochemical plating, chemical vapor deposition, anodic oxidation, ion implantation and deposition, glow discharge plasma treatment, etc., or atomic layer deposition, paint coating technology, ceramic coating technology, chemical conversion film technology, cladding technology, color dyeing and staining technology, surface penetration expansion technology, welding and cladding, laser alloying technology, etc., and may also be a combination of one or more surface modification methods. For the inner and outer surfaces of the medical implant device with a three-dimensional shape or the medical implant device with a groove, the preferred coating method for realizing a full wrapping of the surface coating is atomic layer deposition and electrochemical plating, or a physical or chemical vapor deposition method in which the carrier table and the sample can be rotated to realize a full wrapping.

In some of embodiments, the substrate may be subj ected to the steps of acid washing, electrochemical polishing, and surface activation treatment in sequence before preparing the first coating. The purpose of acid washing is to remove surface cutting debris, heat-affected layer and contaminants. The purpose of electrochemical polishing is to remove the oxidized layer, to obtain a bright and flat surface, to reduce surface roughness, and thus to reduce the risk of thrombosis and tissue reaction after implantation of the medical device, and to obtain the dimension in accordance with the design requirements of the stent. The purpose of surface activation treatment is to remove the passivation film on the surface of the stent and to expose the fresh substrate material.

In some embodiments, the substrate is a tubular substrate. Taking a cardiovascular stent in medical implant device as an example, the preparation method includes the following steps:
Forming a substrate into a tubular member, cutting the tubular member to form a wavy rod, and depositing a first coating on the wavy rod.

Further, the preparation method may also include the following steps:

After forming the wavy rod and prior to depositing the first coating, digging a recess in the wavy rod. As such, the first coating is deposited after the recess is dug, such that the first coating is also formed with a recess, which can be used for carrying medicine.

In some embodiments, the preparation method may also include a step of forming the second coating on the first coating. When the first coating is formed with the medicine-carrying recess, the second coating is also correspondingly formed with the recess at that position, such that the outermost coating is also formed with the recess, which is used for carrying medicine.

According to the aforementioned preparation method of the medical device, a three-dimensional medical device with full coverage of the inner and outer surfaces, uniform thickness, and excellent bonding force can be obtained, which ensures the biosafety of vascular stents and other medical implantable devices, and meanwhile improves the corrosion resistance. The method can be used for the preparation of vascular stents and other medical implant devices, and can prolong the service life of the vascular stents and other medical implant devices in vivo.

In order to make the purpose, the technical solution and the advantages of the present disclosure more concise and clearer, the present disclosure is illustrated with the following specific embodiments, but the present disclosure is by no means limited to these embodiments. The following described embodiments are only the preferable embodiments of the present disclosure and may be used to describe the present disclosure, and are not to be construed as limiting the scope of the present disclosure. It should be noted that any modification, equivalent replacement, and improvement, etc. made within the spirit and principle of the present disclosure shall be included in the protection scope of the present disclosure.

### 1. Preparing the vascular stents of examples 1-20

Cutting, acid washing, heat treatment and polishing of alloy tubular substrate material in sequence;
the sample is then pretreated, degreased, acid washed, and activated by etching;
the first coating of nanometer size with the desired thickness is obtained by electrochemical plating. The electrochemical plating includes liquid-phase mass transfer, electrochemical reaction, and electro-crystallization, and the vascular stent is obtained. The conditions of the electrochemical plating are as follows: a current of 0.05 A, a plating time of 10 min, a stirring rate of 800 rpm, and a temperature of 45°C.

The substrate material of the vascular stent and the material of the first coating of the examples are shown in Table 2 below:

**Table 2**

| Vascular stent | Substrate material | The first coating | |
|---|---|---|---|
| | | The inner layer, thickness | The outer layer, thickness |
| Example 1 | L605 | Cr, 100 nm | Cr₂O₃, 150 nm |
| Example 2 | | Cr, 200 nm | CrN, 80 nm |
| Example 3 | | Cr, 20 nm | CrC, 200 nm |
| Example 4 | | / | Cr₂O₃, 250 nm |
| Example 5 | | / | CrN, 300 nm |
| Example 6 | | / | CrC, 400 nm |
| Example 7 | | / | Cr, 500 nm |
| Example 8 | | / | Cr, 350 nm (passivated) |
| Example 9 | L605 | W, 150 nm | WC, 100 nm |
| Example 10 | | W, 10 nm | WO₃, 250 nm |
| Example 11 | | / | WC, 80 nm |
| Example 12 | | / | WO₃, 350 nm |
| Example 13 | | / | W, 200 nm |
| Example 14 | MP35N | Mo, 50 nm | MoSi₂, 100 nm |
| Example 15 | | / | Mo, 500 nm |
| Example 16 | 316L | Cr, 10 nm | Cr₂O₃, 300 nm |
| Example 17 | | CrN, 400 nm | |
| Example 18 | PtCr alloy | Pt, 200 nm | PtIr, 150 nm |
| Example 19 | | / | Pt, 500 nm |
| Example 20 | | Cr, 150 nm | Cr₂O₃, 50 nm |

The step of passivation treatment in Example 8 is: immersing the stent in a 50 wt% nitric acid solution and passivating at a temperature of 25°C for 50 min.

A comparison of the performance of Examples 1-2 and Examples 4-5 and Example 7 is shown in Table 3 below:

**Table 3**

| Vascular stent | The material of the first coating | Advantages of the first coating |
|---|---|---|
| Example 1 | Cr+Cr₂O₃ | The best chemical stability; Cr transition layer between the substrate and Cr2O3 to enhance the bonding force between the two, excellent compactness and good ductility of the coating. |
| Example 2 | Cr+CrN | Having a transition layer, CrN as an inorganic metal-ceramic phase, very high hardness and great brittleness, and relative high risk of coating cracking after deformation of the stent; it is recommended to strictly control the process and the ratio of coating thickness. |
| Example 4 | Cr₂O₃ | Excellent chemical stability, no pure Cr transition layer between the substrate, the coordination of deformation with the substrate is slightly worse than Example 1, large possibility of cracking after deformation. |
| Example 5 | CrN | Excellent chemical stability, no pure Cr transition layer between the substrate, the coordination of deformation with the substrate is slightly worse than Example 2, the possibility of cracking after deformation is large than Example 4, the thickness should be strictly controlled. |
| Example 7 | Cr | Excellent ductility; excellent bonding force; relatively good high-power scanning electron microscope micro-morphology; bright silver white; natural passivation in air, slightly poor chemical stability. |

### Example 21: The vascular stent with the recess.

The vascular stent of Example 21 is substantially the same as the vascular stent of Example 1, the difference between them lies in that, the substrate used for the vascular stent of Example 21 is provided with the recess, and the correspondingly formed first coating is also provided with the recess.

**Table 4**

| Vascular stent | Substrate material | The first coating | |
|---|---|---|---|
| | | The inner layer, thickness | The outer layer, thickness |
| Example 21 | Cobalt-based alloy L605 | Cr, 50 nm | Cr₂O₃, 150 nm |

As can be seen from FIG. 1 and Table 4, the present disclosure is also applicable to the vascular stent with the recess, the recess is uniformly and completely covered with the first coating, and there is no large-area crack falling off and cracking of the functional coating after the extreme expansion of the stent, the corrosion resistance test meets the requirements, and the results of the ion dissolution and biological experiments are good.

### Example 22: An implanted electrode for a heart pacemaker containing a second functional coating.

The implanted electrode of Example 22 has substantially the same substrate material and first coating as the vascular stent of Example 14, the difference between them lies in that, the implanted electrode of Example 22 further has the silicon layer as the second coating on top of the outer layer of MoSi₂. Detailed materials are shown in Table 5 below:

**Table 5**

| Implanted electrode for a heart pacemaker | Substrate material | The first coating | | The second coating |
|---|---|---|---|---|
| | | The inner layer, thickness | The outer layer, thickness | The outermost layer, thickness |
| Example 22 | MP35N | Mo, 30 nm | MoSi₂, 50 nm | Si, 80 nm |

### Example 23: An artificial knee joint.

| Artificial knee joint | Substrate material | The first coating | The second coating |
|---|---|---|---|
| | | The inner layer, thickness | The outermost layer, thickness |
| Example 23 | Cobalt-chromium-molybdenum alloy ASTM F75 | Cr, 100 nm | Cr₂O₃, 300 nm |

### Example 24: A metal bone nail.

| Metal bone nail | Substrate material | The first coating, thickness |
|---|---|---|
| Example 24 | Stainless steel 316L | CrN, 500 nm |

### Comparative Example

The substrate materials of the vascular stents and the materials of the first coating of the comparative examples are shown in Table 6 below:

**Table 6**

| | | | |
|---|---|---|---|
| Vascular stent | Substrate material | The first coating | |
| | | The inner layer, thickness | The outer layer, thickness |
| Comparative Example 1 | NiTi alloy | Ti, 80 nm | TiO₂, 120 nm |
| Comparative Example 2 | NiTi alloy | Ti, 80 nm | TiN, 150 nm |
| Comparative Example 3 | NiTi alloy | / | Ti, 160 nm |
| Comparative Example 4 | MP35N | / | TiN, 100 nm |
| Comparative Example 5 | MP35N | / | TiN, 200 nm |
| Comparative Example 6 | L605 | / | TiN, 100 nm |
| Comparative Example 7 | 316L | / | TiN, 100 nm |
| Comparative Example 8 | PtCr | / | TiN, 100 nm |
| Comparative Example 9 | L605 | / | Teflon, 200 nm |

### Performance Test 1: Ductility of the stent during use in the presence of large plastic deformation.

The separation of functional coating is related to the properties of the material itself, the separation at the interface (e.g., falling off of the coating and large-area cracking) is mainly determined by the bonding strength (i.e., bonding force) between the functional coating/the substrate.

In accordance with standard medical device implantation procedures, the vascular stent is first compressed onto a balloon, and expanded to the nominal size of the stent with a filler after simulated human traversing three times in a PBS solution at 37°C. For the vascular stent of the embodiments of the present disclosure, on the basis of expanding to the nominal size of the stent, the stent was further expanded with a post-expansion balloon to increase the pressure to continuously post-expand the stent to reach the extreme post-expansion size of the stent, and the bonding performance of the coating on the stent of the present disclosure is verified under this extreme condition.

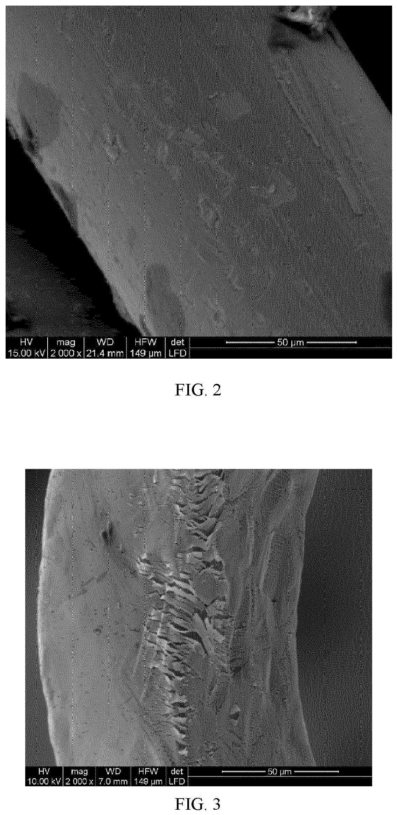

The NiTi alloy in the Comparative Example 1-3 is nickel-titanium wire, and the products made are nickel-titanium wire knitted products, which have super-elasticity but no plastic deformation properties, and do not deform plastically during implantation, so the requirement for the coating bonding force is low. Further, the product made by Comparative Example 2 is subjected to simulated implantation, and it was verified that a coating falling off phenomenon did occur on the product during the simulated implantation, as shown in the SEM image of FIG. 2. It can be known from this that the coating falling off phenomenon on the NiTi alloy occurred just during elastic deformation. Comparative Example 1, Comparative Example 3 and Comparative Example 2 made products with similar properties, i.e., poor bonding force between their coatings and the substrate.

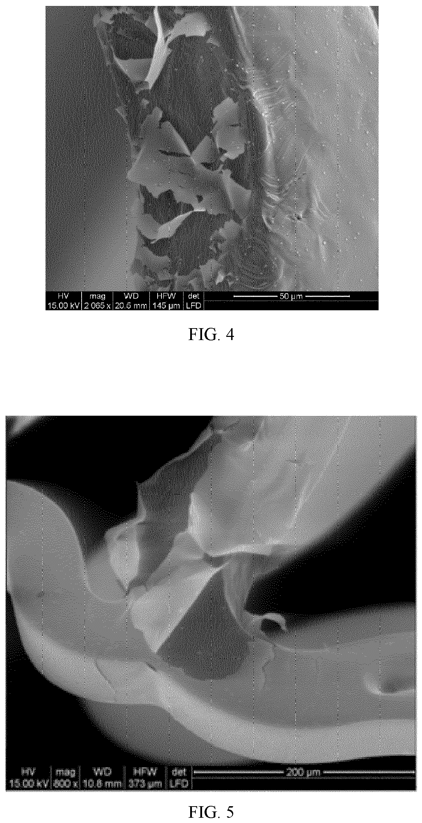

As can be seen from Comparative Example 2 and Comparative Example 4-8, Ti elemental coating and TiN composite coating are not applicable to the surface of materials other than nickel-titanium alloys, such as L605, MP35N, 316L and PtCr, and the like, which require the substrate to be capable of plastic deformation, and the bonding force is poor. The Ti alloy element is used as impurity element in L605, MP35N, 316L and PtCr, and their Ti alloy content is low, not more than 1%, and their elemental or composite coatings have poor bonding force on the substrate, and cracking or even severe coating peeling occurs after the stent was expanded. FIG. 3 and FIG. 4 are the SEM images of the vascular stents made by Comparative Example 4-5, which show cracking or even serious coating peeling after the stents were expanded to a nominal size, respectively. The results of this experiment also show that when the stent is expanded to the nominal size, the tendency of cracking increases with the larger coating thickness under the same deformation condition.

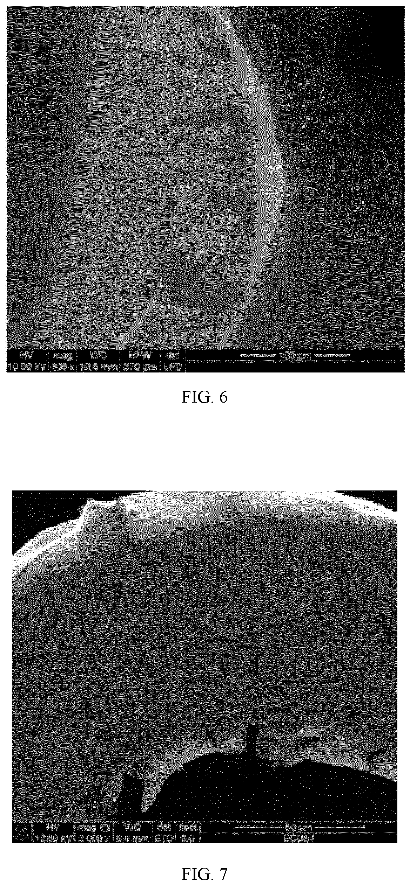

FIG. 5 is the scanning electron microscope image of the stent product made in Comparative Example 6; FIG. 6 is the scanning electron microscope image of the stent product made in Comparative Example 7 expanded to nominal size; FIG. 7 is the scanning electron microscope image of the stent product made in Comparative Example 8 expanded to extreme post-expansion size. As can be seen, the substrate of the stent products of Comparative Example 6-8 does not contain the Ti alloy element, and only the substrate MP35N contains trace impurity element of Ti (<1%), while the coatings are all TiN. Therefore, the substrate and the coating material do not have the same alloy element, and there exists a relatively large self-corrosive potential difference, and the coating bonding force is relatively poor. The stent obtained in Comparative Example 6 is not expanded, and the coating is found to be peeled off in a large area only after ultrasonic cleaning. After the stent obtained in Comparative Example 7 is post-expanded to nominal size, the coating in a large-area is peeled off. After the stent obtained in Comparative Example 8 is post-expanded, the coating shows a large-area of cracking, and has a risk of coating falling off. During stent implantation and post-expansion, if the coating falls off, it will lead to serious consequences of thrombosis and death.

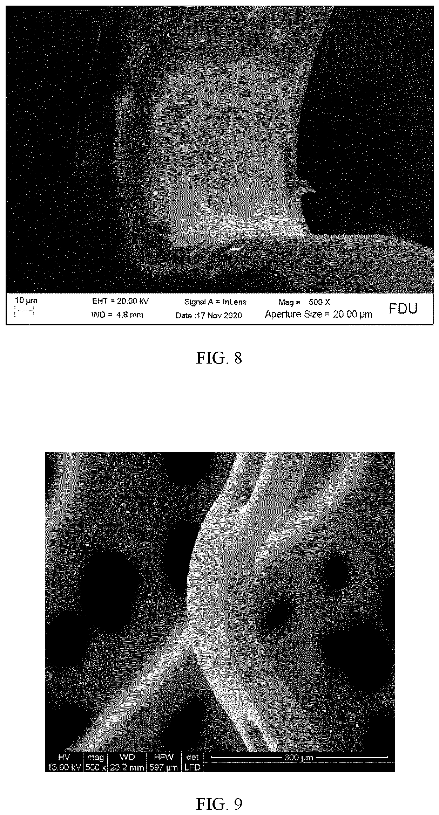

The stent made in Comparative Example 9 had not yet undergone plastic deformation, and after ultrasonic cleaning only, after PBS simulated traversal (i.e., before nominal size expansion) the coating is found to be large-area broken, and its SEM is shown in FIG. 8.

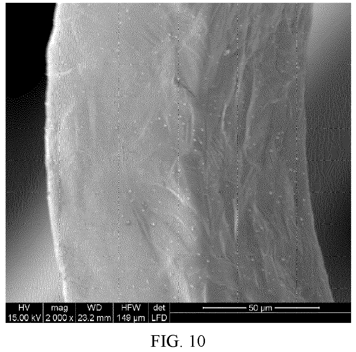

The surface morphology of the stent made in Example 4 expanded to nominal size is shown in FIG. 9; and the surface morphology of the stent rod made in Example 8 under a condition of the extreme straightened expansion deformation is shown in FIG. 10. The coating and the substrate are very firmly bonded together, and the coating does not crack or fall off, which proves that the coating of the present disclosure has excellent bonding and ductility with the substrate. This shows that the stent made by the present disclosure can meet the requirements of large plastic deformation and has excellent reliability.

### Performance Test 2: Dissolution of nickel and cobalt metal ions of the stent under simulated use condition.

Whether the functional coating can effectively remove, isolate or avoid the dissolution of nickel and cobalt sensitized and carcinogenic alloy elements or not can be determined using an atomic emission spectroscopy quantitative analysis test method (ICP-MS).

For the stent products made in the above examples, take an uncoated sample as a control sample, while a nickel ion dissolution of a nickel-free alloy Biodur 108 is taken as a blank control sample. The total surface area of each stent is 12 cm², and the leachate is taken after 1 week of leaching in PBS incubation solution at 50°C, results of atomic emission spectrometry quantitative analysis tests of contents of nickel and cobalt metal ions in the leachate tested by ICP-MS, are shown in Table 7.

**Table 7**

| Group | Substrate material | The first coating | | The result of atomic emission spectrometry quantitative analysis test (µg/mL) | |
|---|---|---|---|---|---|
| | | The inner layer | The outer layer | Co | Ni |
| Blank Control | Nickel-free alloy Biodur 108 | / | / | / | <0.002 |
| L605 uncoated | | / | / | 0.045 | 0.014 |
| Example 1 | L605 | Cr | Cr₂O₃ | 0.005 | 0.003 |
| Example 2 | | Cr | CrN | 0.006 | 0.005 |
| Example 3 | | Cr | CrC | 0.007 | 0.008 |
| Example 4 | | / | Cr₂O₃ | 0.005 | 0.003 |
| Example 5 | | / | CrN | 0.006 | 0.005 |
| Example 6 | | / | CrC | 0.007 | 0.008 |
| Example 7 | | / | Cr | 0.008 | 0.006 |
| Example 8 | | / | Cr (passivated) | <0.002 | <0.002 |
| Example 9 | | W | WC | 0.01 | 0.006 |
| Example 10 | | W | WO₃ | 0.008 | 0.004 |
| Example 11 | | / | WC | 0.01 | 0.006 |
| Example 12 | | / | WO₃ | 0.008 | 0.004 |
| Example 13 | | / | W | <0.002 | <0.002 |
| MP35N uncoated | | / | / | 0.06 | 0.023 |
| Example 14 | MP35N | Mo | MoSi₂ | 0.009 | 0.005 |
| Example 15 | | / | Mo | <0.002 | <0.002 |
| 316L uncoated | | / | / | / | 0.017 |
| Example 16 | 316L | Cr | Cr₂O₃ | / | 0.003 |
| Example 17 | | / | CrN | / | 0.005 |
| PtCr alloy uncoated | | / | / | / | 0.01 |
| Example 18 | PtCr alloy | Pt | PtIr | / | 0.007 |
| Example 19 | | / | Pt | / | <0.002 |
| Example 20 | | Cr | Cr₂O₃ | / | 0.003 |
| Example 22 | MP35N | Mo | MoSi₂ | 0.006 | 0.002 |
| | | The second coating | Si | | |
| Example 23 | Cobalt-chromium-molybdenum alloy ASTM F75 | Cr | Cr₂O₃ | 0.005 | 0.002 |
| Example 24 | Stainless steel 316L | / | CrN | / | 0.005 |
| NiTi alloy uncoated | | / | / | / | 0.04 |
| Comparative Example 1 | NiTi alloy | Ti | TiO₂ | / | 0.028 |
| Comparative Example 2 | | Ti | TiN | / | 0.03 |
| Comparative Example 3 | | / | Ti | / | 0.025 |

The results of atomic emission spectroscopy quantitative analysis test ICP-MS ion dissolution show that the coating on the vascular stent of the present disclosure can effectively reduce the dissolution of nickel, cobalt and other harmful ions, and the reduction of nickel ions can reach a ppm level; in particular, for vascular implant stents with cobalt-based alloys such as L605, MP35N, and ASTM F75, the reduction of their cobalt ions dissolution is especially obvious. For the NiTi alloys in Comparative Example 1-3, the first coating results in a decrease of the dissolution of nickel ion, and in addition, the Ti ion of about 0.02 µg/mL is detected in the solution, exacerbating the risk of biosafety ions dissolution in implant medical devices.

As can be seen from the provided scanning electron microscope images that, the coatings of the stent products of Comparative Example 4-9 have a large number of cracking or falling off, the coating cannot play the role of blocking harmful ions dissolved, the dissolution performance of nickel and cobalt metal ions and subsequent corrosion resistance are not investigated here.

As can be seen from the scanning electron microscope images that, in the stent products of Comparative Examples 1 to 3, a certain degree of coating falling off also occurred, i.e., the bonding force of their coatings and the substrate is poor, and therefore their blocking effect on the dissolution of nickel ions is very limited, and therefore their corrosion resistance potential are not investigated subsequently.

Comparison of Example 22 with Example 14 shows that the presence of the second coating further reduces the dissolution of nickel and cobalt metal ions of the stent under simulated use conditions.

### Performance Test 3: The corrosion resistance of the stent.

According to YYT 0695-2008 Standard test method for conducting cyclic potential dynamic polarization measurements to determine the corrosion susceptibility of small implant devices (ASTM F 2129-06), the test conditions are 37°C and PBS solution; the corrosion resistance of each of the above examples is tested in accordance with the standard test method of TC, and the uncoated stent sample is used as a control sample. The resulting test results are shown in Table 8:

**Table 8**

| Group | Substrate material | The first coating | | Self-corrosion potential E_{corr} vs SCE (mV) | Breakdown potential E_{b} vs SCE (mV) | Protection potential Ep vs SCE (mV) |
|---|---|---|---|---|---|---|
| | | The inner layer Thickness | The outer layer Thickness | | | |
| L605 uncoated | | / | / | 186 | 633 | 605 |
| Example 1 | L605 | Cr | Cr₂O₃ | 197 | 803 | 780 |
| Example 2 | | Cr | CrN | 173 | 756 | 702 |
| Example 3 | | Cr | CrC | 176 | 708 | 645 |
| Example 4 | | / | Cr₂O₃ | 200 | 799 | 778 |
| Example 5 | | / | CrN | 170 | 753 | 698 |
| Example 6 | | / | CrC | 178 | 706 | 646 |
| Example 7 | | / | Cr | 208 | 726 | 675 |
| Example 8 | | / | Cr (passivated) | 202 | 806 | 750 |
| Example 9 | | W | WC | 170 | 791 | 752 |
| Example 10 | | W | WO₃ | 179 | 761 | 723 |
| Example 11 | | / | WC | 172 | 795 | 750 |
| Example 12 | | / | WO₃ | 180 | 758 | 721 |
| Example 13 | | / | W | 186 | 735 | 682 |
| MP35N uncoated | | / | / | 150 | 612 | 548 |
| Example 14 | MP35N | Mo | MoSi₂ | 169 | 710 | 653 |
| Example 15 | | / | Mo | 173 | 698 | 649 |
| 316L uncoated | | / | / | 323 | >800 | / |
| Example 16 | | Cr | Cr₂O₃ | 199 | 802 | 782 |
| Example 17 | | / | CrN | 171 | 756 | 701 |
| PtCr alloy uncoated | | / | / | 518 | >800 | / |
| Example 18 | PtCr alloy | Pt | PtIr | 505 | >800 | |
| Example 19 | | / | Pt | 530 | >800 | / |
| Example 20 | | Cr | Cr₂O₃ | 198 | 801 | 781 |
| Example 22 | MP35N | Mo, 30nm | MoSi₂, 50nm | 168 | 798 | / |
| | | The second coating | Si, 80 nm | 155 | 825 | / |
| Example 23 | Cobalt-chromiu m-molybde num alloy ASTM F75 | Cr | Cr₂O₃ | 198 | 802 | 780 |
| Example 24 | Stainless steel 316L | / | CrN | 172 | 755 | 700 |
| NiTi alloy uncoated | | / | / | 157 | >800 | / |
| Comparative Example 1 | NiTi alloy | Ti | TiO₂ | - | >800 | / |
| Comparative Example 2 | | Ti | TiN | - | >800 | / |
| Comparative Example 3 | | / | Ti | - | >800 | / |

It is worth noting that the self-corrosion potentials in Table 8 are tested with the stent samples as objects, and the stent samples are made by the substrate and the coatings wrapping on the substrate, so the measured self-corrosion potentials can be considered to be the self-corrosion potentials of the outermost layer of the coating on the substrate. Taking L605 uncoated and Examples 1-8 as examples, the self-corrosion potential of L605 uncoated in Table 8 is the self-corrosion potential of the substrate, and the self-corrosion potentials of Examples 1-8 in Table 8 can be considered to be the self-corrosion potentials of Cr₂O₃, CrN, CrC, Cr₂O₃, CrN, CrC, Cr, and Cr (passivated), respectively, and based on which the differences of the self-corrosion potential between any two adjacent layers of layer structures of the stent samples can be obtained.

As Class III implant medical devices, vascular stents and other medical implant devices should have excellent corrosion resistance, requiring that the breakdown potential should be ≥600mV and the protection potential should be ≥300mV The test results show that the corrosion resistance of all coated stent samples is better than that of uncoated stent control samples, which meets the use requirements of the product's corrosion resistance that the breakdown potential should be ≥600mV and the protection potential should be ≥300mV. When the protection potential in Table 8 is "/", it means infinity.

According to the comparison of Example 7 and Example 8, when the first coating is the elemental metal layer and is used as the outer layer, the breakdown potential of the stent product is increased to 806 mV from 726 mV prior to the unpassivated treatment after the elemental metal layer is subjected to the passivation treatment.

Compared the corrosion resistance of Comparative Example 1-3 with the corrosion resistance of NiTi alloy without coating, there is no obvious difference, i.e., it reflects the nature of the substrate material itself, and it is presumed that the bonding force between the coating and the substrate is poor and certain falling off phenomena have occurred. And, the coating of Comparative Example 1-3 has occurred certain falling off phenomenon, resulting in its inability to effectively isolate or significantly reduce the dissolution of the nickel ion in the substrate.

### Performance Test 4.

For each of the above examples, samples made by the examples are taken for cytotoxicity, hemolysis and coagulation biology tests to further determine the biosafety of the functional coating.

The results show that the stent samples made by the examples all meet the biosafety requirements of cell viability greater than or equal to 70%, hemolysis rate <5%, and four coagulation tests p>0.05.

Each of the technical features of the above-mentioned embodiments may be combined arbitrarily, to simplify the description, not all the possible combinations of each of the technical features in the above embodiments are described, however, all of the combinations of these technical features should be considered as within the scope of this disclosure, as long as such combinations do not contradict with each other.

The aforementioned embodiments merely represent several embodiments of the present disclosure, and the description thereof is specific and detailed, but it should not be construed as limiting the scope of the present disclosure. It should be noted that a plurality of variations and modifications may be made by those skilled in the art without departing from the scope of this disclosure, which are all within the scope of protection of this disclosure. Therefore, the protection scope of this disclosure shall be subject to the appended claims.

## Claims

1. A medical device, comprising a substrate and a first coating wrapping the substrate, wherein the substrate contains a metal element X with a content of ≥5wt%, the first coating comprises at least one of an elemental metal layer of the metal element X, an alloy layer of the metal element X, and a metal-ceramic layer of the metal element X, and the first coating does not contain nickel and cobalt.

2. The medical device according to claim 1, wherein the first coating is a stack layer formed by the elemental metal layer and at least one of the alloy layer and the metal-ceramic layer; in the first coating, the elemental metal layer is an inner layer close to the substrate, at least one of the alloy layer and the metal-ceramic layer is an outer layer away from the substrate.

3. The medical device according to claim 1, wherein the elemental metal layer is a passivated metal coating when the first coating is the elemental metal layer of the metal element X.

4. The medical device according to claim 1, wherein the elemental metal layer is located between the substrate and the metal-ceramic layer when the first coating is the stack layer formed by the elemental metal layer and the metal-ceramic layer.

5. The medical device according to claim 1, wherein the elemental metal layer is located between the substrate and the alloy layer when the first coating is the stack layer formed by the elemental metal layer and the alloy layer.

6. The medical device according to claim 1, wherein a material of the metal-ceramic layer is at least one of oxide, carbide, nitride, silicide, and boride of the metal element X.

7. The medical device according to claim 1, wherein the metal element X is Cr, W, Mo or any one of noble metal element.

8. The medical device according to claim 1, wherein the content of the metal element X in the substrate ranges from 7 wt% to 60 wt%.

9. The medical device according to claim 1, wherein the substrate is a substrate capable of plastic deformation.

10. The medical device according to claim 1, wherein a material of the substrate is selected from the group consisting of stainless steel, cobalt-based alloy, titanium alloy, noble metal alloy, and nickel-titanium shape memory alloy.

11. The medical device according to claim 1, wherein the metal element X is one of Cr, W, Mo or Pt.

12. The medical device according to claim 1, wherein the first coating contains the metal-ceramic layer, the medical device further comprises a second coating provided on the metal-ceramic layer;
wherein the metal-ceramic layer is a carbide of the metal element X, and the second coating is a carbon layer.

13. The medical device according to claim 1, wherein the first coating contains the metal-ceramic layer, the medical device further comprises a second coating provided on the metal-ceramic layer;
wherein the metal-ceramic layer is a silicide of the metal element X, and the second coating is a silicon layer.

14. The medical device according to any one of claims 1 to 13, wherein a difference of self-corrosion potential between any two adjacent layers of layer structures with different materials of the medical device is within 100 mV.

15. The medical device according to any one of claims 1 to 13, wherein thicknesses of the elemental metal layer, the alloy layer, and the metal-ceramic layer range from 10 nm to 500 nm.

16. The medical device according to any one of claims 1 to 13, wherein the metal element X is not element titanium.

17. The medical device according to any one of claims 1 to 13, further comprising a medicine active layer located on a surface of the first coating.

18. The medical device according to claim 17, wherein the first coating is formed with a medicine-carrying recess, and the medicine active layer is located within the medicine-carrying recess.
